# EUROPÄISCHE PATENTANMELDUNG

(11) **EP 1 078 909 A1**
(43) Veröffentlichungstag der Anmeldung: **28.02.2001**
(21) Anmeldenummer: 00115210.7
(22) Anmeldetag: 13.07.2000
(51) Int. Cl.: C07C 45/79, C07C 45/78, C07C 45/82, C07C 49/08

(54) **Verfahren zur Herstellung von methanolarmem Aceton**

(30) Priorität: 24.08.1999 DE 19940008
(71) Anmelder: Phenolchemie GmbH & Co. KG, 45966 Gladbeck (DE)
(72) Erfinder: Gerlich, Otto, Dr., 45966 Gladbeck (DE); van Barneveld, Heinrich, Dr., 46244 Bottrop (DE); Jordan, Wilfried, Dr., 46282 Dorsten (DE)
(74) Vertreter: Olbricht, Gerhard, Dr.

(57) **Zusammenfassung**

Bei der Herstellung von Phenol und Aceton nach dem Hock-Verfahren fallen Acetonströme an, die in nicht vernachlässigbarem Umfang Methanol und Wasser enthalten und je nach Kundenanforderung gereinigt werden müssen. Eine Molekularsiebbehandlung zur Abtrennung von Methanol und Wasser benötigt recht große Molekularsiebmengen, um vorgegebene Methanolgrenzwerte zu erreichen, wodurch die Wirtschaftlichkeit des Verfahrens eingeschränkt ist.

Erfindungsgemäß wird der Molekularsiebbehandlung ein Trocknungsschritt vorangestellt, wodurch die benötigte Molekularsiebmenge unerwartet stark reduziert werden kann.

## Beschreibung

Die Erfindung betrifft ein Verfahren zur Herstellung von methanolarmem Aceton aus Aceton, das nach dem Hock-Verfahren gewonnen wurde und 200 Gew.-ppm oder mehr Methanol sowie bis zu 0,3 Gew.-% Wasser enthält, durch Molekularsiebbehandlung.

Das Prinzip der kombinierten Herstellung von Phenol und Aceton nach dem Hock-Verfahren beruht auf der Oxidation von Cumol zu Cumolhydroperoxid und der anschließenden säurekatalysierten Spaltung des Cumolhydroperoxids zu Phenol und Aceton. Das bei dieser Spaltreaktion entstehende Reaktionsprodukt wird anschließend zur Gewinnung von Phenol und Aceton aufgearbeitet. Bei dieser Aufarbeitung gilt es, zugleich verschiedene Verunreinigungen, die durch Nebenproduktbildung teils schon aus der Cumolherstellung herrühren (z. B. durch Dimerisierung des Propens), teils aber auch erst im Zuge des Hock-Verfahrens entstehen, abzutrennen. Zu diesen Verunreinigungen gehört neben z. B. Aldehyden, Estern, Säuren und anderen Alkoholen auch Methanol, das vorwiegend bei der Cumoloxidation und Spaltung von Cumolhydroperoxid als Nebenprodukt gebildet wird. Methanol begleitet bei der Aufarbeitung des Produktstroms weitestgehend das Produkt Aceton, da es mit dem Aceton ein Azeotrop bildet, dessen Siedepunkt von 55,5 °C praktisch mit dem Siedepunkt von reinem Aceton bei 56,2 °C übereinstimmt, so daß eine Abtrennung des Methanols vom Aceton durch herkömmliche, rein destillative Reinigungsstufen nicht möglich ist. Im Zuge der Aufarbeitung des Spaltproduktes fallen daher schließlich Acetonströme an, die teilweise zwar bereits eine Permanganatstandzeit (Indiz für reduzierende Bestandteile) von über 2 h (nach ASTM D 1363) und einen Benzolgehalt von weniger als 50 Gew.-ppm aufweisen, die aber noch bis zu 0,3 Gew.-% Wasser und von ca. 200 bis hin zu einigen Tausend Gew.-ppm Methanol enthalten und daher nicht in jedem Fall bereits den Kundenanforderungen gerecht werden. Der relativ hohe Wassergehalt in diesen Strömen kommt im wesentlichen dadurch zustande, daß als Säurekatalysator bei der

Cumolhydroperoxidspaltung zumeist (wäßrige) Schwefelsäure verwendet wird und daß sich als Nebenprodukt in nennenswertem Umfang Dimethylphenylcarbinol bildet, welches teilweise zu α-Methylstyrol dehydratisiert. Auch bei Aufarbeitungsprozessen, in denen zur Entfernung von Acetaldehyd aus Aceton ferner eine Wäsche mit Natronlauge vorgesehen ist, bestimmt letztlich stets die Trennleistung der nachgeschalteten Reinacetonkolonne den Wassergehalt von bis zu 0,3 Gew.-% in den Acetonströmen. Um den stetig steigenden Kundenspezifikationen für das Produkt Aceton zu entsprechen, bedürfen diese Acetonströme zumindest einer Verringerung des Methanolgehalts.

Aus dem US-Patent 2,581,789 ist bekannt, daß methanolhaltiges Aceton unter Einsatz eines Schleppmittels destillativ von Methanol gereinigt werden kann. Es wird der Einsatz von Hexan zur Abtrennung von Methanol aus Aceton beschrieben, wobei als Kopfprodukt ein ternäres Azeotrop Hexan/Methanol/Aceton isoliert wird, während als Sumpfprodukt Aceton mit einem Gehalt an Methanol von weniger als 500 Gew.-ppm anfällt.

Das US-Patent 4,501,645 beschreibt ferner den Einsatz von aliphatischen Ketonen der Kohlenstoffkettenlängen C₄ bis C₁₁ als Extraktionsmittel für die Entfernung von Methanol aus Aceton durch Extraktivdestillation.

Aus dem tschechoslowakischen Patent 268 005 ist weiterhin bekannt, daß sich auch durch Zugabe von Wasser eine Abtrennung des Methanols aus Aceton erreichen läßt. Hierbei wird durch die Zuführung des Wassers auf den Kolonnenkopf die stark assoziierende Wirkung zwischen Alkohol und Wasser genutzt, um den Alkohol Methanol in der Blase anzureichern, während der nicht assoziierte Bestandteil, nämlich das Aceton, als Destillat übergeht.

Diesen bekannten Verfahren zur Gewinnung von methanolarmem Aceton ist gemeinsam, daß sie zusätzlich stets produktionsfremde Hilfsstoffe wie z. B. zur Extraktivdestillation einsetzen, die den Bearbeitungsaufwand deutlich erhöhen.

DE-AS 1 793 516 lehrt, daß Aceton mit u. a. einem Methanolgehalt von ca. 500 bis 600 Gew.-ppm von Wasser und Methanol gereinigt werden kann, indem man es einer Molekularsiebbehandlung mittels eines Molekularsiebes mit einem Porendurchmesser von 0,4 oder 0,5 nm unterwirft. Unter Einhaltung einer definierten Verweilzeit von 5 Minuten und unter Verwendung von z. B. 20 Gew.-% Molekularsieb bezogen auf das eingesetzte Aceton ist es so möglich, sowohl Methanol und Wasser zu entfernen als auch eine unerwünschte Diacetonalkoholbildung zu unterdrücken.

Die Abtrennung von Methanol mittels Molekularsiebbehandlung ist zwar deutlich einfacher zu handhaben als die zuvor genannten Verfahren, sie stellt jedoch einen Prozeßschritt dar, der insbesondere bei kontinuierlicher Betriebsweise wegen der nach DE-AS 1 793 516 benötigten Molekularsiebmenge und den damit verbundenen recht großen Apparaten einen nicht zu vernachlassigenden Aufwand mit sich bringt.

Damit stellt sich die Aufgabe, ein einfaches Verfahren zur Herstellung von methanolarmem Aceton aus Aceton, das nach dem Hock-Verfahren gewonnen wird und die obengenannten Spezifikationen besitzt, zu finden, das ohne den Zusatz produktionsfremder Stoffe arbeitet und eine verbesserte Wirtschaftlichkeit gewährleistet.

Diese Aufgabe wird erfindungsgemäß gelöst gemäß Patentanspruch 1 durch ein Verfahren zur Herstellung von methanolarmem Aceton aus Aceton, das nach dem Hock-Verfahren gewonnen wurde und 200 Gew.-ppm oder mehr Methanol sowie bis zu 0,3 Gew.-% Wasser enthält, durch Molekularsiebbehandlung, das dadurch gekennzeichnet ist, daß vor der Molekularsiebbehandlung Wasser aus dem Aceton abgetrennt wird.

Überraschenderweise wurde gefunden, daß sich durch eine Wasserabtrennung vor der Molekularsiebbehandlung der Molekularsiebbedarf für eine vorgegebene Verringerung des Methanolgehalts im Aceton unerwartet und überproportional stark gegenüber der Lehre von DE-AS 1 793 516 verringern läßt. Der insbesondere auch apparative Aufwand dieses Prozeßschrittes sinkt damit beträchtlich. Dieser unerwartete Effekt ist nach eigenen Untersuchungen im wesentlichen offenbar darauf zurückzuführen, daß bei dem üblicherweise verwendeten Molekularsiebtyp mit 0,4 bis 0,5 nm Porendurchmesser bei der Adsorption das Wasser das Methanol verdrängt; somit muß zur Abtrennung einer gewissen Methanolmenge gemäß DE-AS 1 793 516 überproportional viel Molekularsieb bereitgestellt werden, das nach Sättigung ersetzt werden muß oder der Regenerierung bedarf. Diese Regenerierung des Molekularsiebs kann erfindungsgemäß z. B. durch Desorption der im Molekularsieb adsorbierten Bestandteile mittels Wasserdampf, Acetondampf oder Dampf anderer geeigneter organischer Verbindungen, heißen Stickstoffströmen oder zeitlich aufeinander folgenden Kombinationen hieraus erfolgen. Da erfindungsgemäß wesentlich weniger Molekularsieb als in DE-AS 1 793 516 benötigt wird, muß auch nur wesentlich weniger Molekularsieb regeneriert oder durch neues ersetzt werden, was einen weiteren Kostenvorteil darstellt. Im Falle der Regeneration mit Wasserdampf sinkt der Dampfbedarf analog zur Gesamtmenge an Molekularsieb beträchtlich. Bei der Regeneration mit heißem Stickstoff sinkt der Wärmebedarf ebenfalls entsprechend. Der Wärmbedarf kann durch geeignete Wärmetauscher minimiert werden. Die Desorption kann auch derart erfolgen, daß der beladene Adsorber zunächst mittels Wasserdampf aufgeheizt wird, während die eigentliche Desorption im heißen Stickstoff-Strom erfolgt.

Wie bereits eingangs beschrieben, fallen im Hock-Prozeß zur Herstellung von Aceton und Phenol im Zuge der Aufarbeitung verschiedene Acetonströme an, die neben bis zu 0,3 Gew.-% Wasser 200 Gew.-ppm oder mehr Methanol enthalten können. Zumeist liegen diese Acetonströme für die erfindungsgemäße Behandlung in flüssiger Form vor; es ist aber auch möglich, gas- bzw. dampfförmige Acetonströme, so wie sie z.B. aus einer Destillationskolonne abdestilliert werden, erfindungsgemäß zu behandeln.

Aus einem derartigen Acetonstrom wird erfindungsgemäß zunächst vor der Molekularsiebbehandlung Wasser abgetrennt. Die Reduzierung des Wassergehalts kann mittels eines aus dem Stand der Technik bekannten Verfahrens der Trocknung von Stoffströmen von Wasser erfolgen, beispielsweise destilativ, extraktiv, durch Auskondensieren des Wassers oder durch Trocknung mit einem unspezifischen Trocknungsmittel wie z. B. Silikagel, wobei der Aggregatzustand des Acetonstroms zu berücksichtigen ist.

Steht kostengünstig - z. B. als Abwärme - Niedertemperaturwärme ausreichender Temperatur (vorzugsweise mit über 100 °C) und Menge zur Verfügung, so erfolgt die Wasserabtrennung insbesondere aus flüssigen Acetonströmen vorzugsweise destillativ. Der Wassergehalt im zu behandelnden Aceton beträgt erfindungsgemäß nach der Behandlung zur Verringerung des Wassergehalts bevorzugt nur noch bis zu 500 Gew.-ppm, besonders bevorzugt nur noch bis zu 100 Gew.-ppm.

Das dermaßen entwässerte Aceton wird nun erfindungsgemäß analog zu DE-AS 1 793 516 einer Molekularsiebbehandlung zur Verringerung des Methanolgehalts unterzogen. Vorzugsweise wird ein Molekularsiebtyp mit 0,4 bis 0,5 nm Porendurchmesser verwendet. Die Behandlung erfolgt üblicherweise zumeist in flüssiger Phase bei Temperaturen bis zu 50 °C, vorzugsweise zwischen 10 °C und 40 °C.

Aufgrund der vorher erfolgten Wasserabtrennung kann nahezu die gesamte Adsorptionskapazität des Molekularsiebes erfindungsgemäß für die Verringerung des Methanolgehalts genutzt werden. Für die bevorzugten Molekularsiebtypen mit 0,4 bis 0,5 nm Porendurchmesser beträgt das Aufnahmevermögen für Methanol und Wasser in der Summe üblicherweise mehr als 4 kg pro 100 kg Molekularsieb. Bei einem Acetonstrom, der beispielsweise 300 Gew.-ppm Methanol und 0,2 Gew.-% Wasser enthält, können daher erfahrungsgemäß beim Verfahren gemäß DE-AS 1 793 516 nur ca. 3 Tonnen dieses Acetons bis auf einen Methanolgehalt von kleiner oder gleich 50 Gew.-ppm pro 100 kg Molekularsieb gereinigt werden. Erfindungsgemäß steht das Adsorptionsvermögen hingegen wegen der vorgeschalteten

Wasserabtrennung bevorzugt nahezu vollständig für das Methanol zur Verfügung. Daher können erfindungsgemäß z. B. 10 Tonnen eines Acetonstroms, dessen Wassergehalt zuvor auf kleiner 200 Gew.-ppm reduziert wurde und der wieder 300 Gew.-ppm Methanol enthält, pro 100 kg Molekularsieb bis auf einen Methanolgehalt von bis zu 50 Gew.-ppm gereinigt werden.

Es wird also wesentlich weniger Molekularsieb benötigt, was sich auch entsprechend in den benötigten Apparaten und den Apparatekosten niederschlägt. Ist die Aufnahmekapazität des verwendeten Molekularsiebes erschöpft, muß es entweder ersetzt oder regeneriert werden. In beiden Fällen ergeben sich wegen der geringeren absoluten Menge an Molekularsieb durch die Energieeinsparung bei der Desorption zusätzliche große Kostenvorteile gegenüber dem herkömmlichen Verfahren nach DE-AS 1 793 156. Die Regenerierung des Molekularsiebes kann ohne Kapazitätsverlust z. B. auch durch einen heißen Stickstoffstrom erfolgen, wobei hier eine Kondensationsvariante mit Kreislaufführung des Stickstoffs oder alternativ eine direkte Regenerierung mit nachgeschalteter Verbrennung z. B. in einer Wärmeträgeranlage möglich ist.

Die Verringerung des Wassergehalts erfolgt erfindungsgemäß bevorzugt durch den im Vergleich zur reinen Molekularsiebbehandlung nach DE-AS 1 793 516 je nach den Gegebenheiten wirtschaftlichsten herkömmlichen Trocknungsprozeß, vorzugsweise destillativ, da geeignete Abwärme häufig bei der destillativen Aufarbeitung des Spaltproduktes aus der Cumolhydroperoxidspaltung kostengünstig vorliegt.

Im Fall eines destillativen Verfahrensschrittes zur Entwässerung des Acetons erfolgt die Methanolabtrennung durch Molekularsiebbehandlung vorzugsweise derart, daß als Kopfprodukt anfallendes wasserarmes Aceton flüssig und/oder gasförmig zunächst mit Molekularsieb zur Methanolabtrennung behandelt wird, bevor zumindest ein Teil des Kopfproduktes der Destillationskolonne erneut als Rücklauf zugeführt wird.

Die erfindungsgemäße Kombination der - vorzugsweise destillativen - Verringerung des Wassergehalts mit einer nachgeschalteten Molekularsiebbehandlung zur Verringerung des Methanolgehalts ermöglicht damit im Vergleich zu den bekannten Verfahren auf sehr einfache und wirtschaftliche Weise die Abtrennung von Methanol und Wasser aus Aceton, wodurch spezifikationsgerechtes methanolarmes Aceton erhalten wird. Das so erhaltene Aceton kann entweder allein oder vereint mit anderen Acetonmengen vermarktet werden. Bevorzugt wird das erfindungsgemäße Verfahren zur Herstellung von Aceton mit einem Methanolgehalt von weniger als 50 Gew.-ppm und einem Wassergehalt von unter 500 Gew.-ppm verwendet.

Das erfindungsgemäße Verfahren kann kontinuierlich oder diskontinuierlich durchgeführt werden. Bevorzugt ist jedoch eine kontinuierliche Fahrweise mit zumindest zwei Molekularsieb enthaltenden Adsorbern, von denen zumindest einer beladen und einer regeneriert wird.

Das erfindungsgemäße Verfahren wird durch die nachfolgenden Beispiele näher erläutert:

### Beispiel 1

Aceton mit einem Gehalt von 300 Gew.-ppm Methanol und 0,2 Gew.-% Wasser wird destillativ zur Reduktion des Wassergehalts behandelt. Es fallen 10 t Aceton mit einem Gehalt von 300 Gew.-ppm Methanol und 200 Gew.-ppm Wasser an, die über 100 kg Molekularsieb mit 0,4 nm Porendurchmesser geleitet werden. Die Temperatur beträgt ca. 30 °C. Es fallen 9,9 t Aceton mit einem Methanolgehalt unter 50 Gew.-ppm und einem Wassergehalt unter 60 Gew.-ppm an, die für die Vermarktung verfügbar sind.

### Beispiel 2

Aus 4 t Aceton mit einem Gehalt von 1 000 Gew.-ppm Methanol und 0,2 Gew.-% Wasser wird destillativ soviel Wasser abgetrennt, daß der Wassergehalt 200 Gew.-ppm beträgt. Diese 4 t Aceton mit 1 000 Gew.-ppm Methanolgehalt und 200 Gew.-ppm Wassergehalt werden anschließend über 100 kg Molekularsieb mit einem Porendurchmesser von 0,5 nm geleitet. Die Temperatur beträgt ca. 30 °C. Es fallen 3,9 t Aceton mit einem Gehalt von weniger als 50 Gew.-ppm Methanol und weniger als 60 Gew.-ppm Wasser an, die direkt für die Vermarktung verfügbar sind.

## Patentansprüche

1. Verfahren zur Herstellung von methanolarmem Aceton aus Aceton, das nach dem Hock-Verfahren gewonnen wurde und 200 Gew.-ppm oder mehr Methanol sowie bis zu 0,3 Gew.-% Wasser enthält, durch Molekularsiebbehandlung,
dadurch gekennzeichnet,
daß vor der Molekularsiebbehandlung Wasser aus dem Aceton abgetrennt wird.

2. Verfahren nach Anspruch 1,
dadurch gekennzeichnet,
daß der Wassergehalt des Acetons auf bis zu 500 Gew.-ppm verringert wird.

3. Verfahren nach Anspruch 2,
dadurch gekennzeichnet,
daß der Wassergehalt des Acetons auf bis zu 100 Gew.-ppm verringert wird.

4. Verfahren nach zumindest einem der vorherigen Ansprüche,
dadurch gekennzeichnet,
daß die Verringerung des Wassergehalts destillativ, extraktiv, durch Auskondensieren oder mittels eines unspezifischen Trocknungsmittels erfolgt.

5. Verfahren nach Anspruch 4,
dadurch gekennzeichnet,
daß die Verringerung des Wassergehalts destillativ mittels Abwärmenutzung erfolgt.

6. Verfahren nach Anspruch 4 oder 5,
dadurch gekennzeichnet,
daß bei destillativer Verringerung des Wassergehalts im Aceton als Kopfprodukt anfallendes wasserarmes Aceton zunächst einer Molekularsiebbehandlung zur Verringerung des Methanolgehalts unterzogen wird, bevor zumindest ein Teil des Kopfproduktes als Rücklauf der Destillationskolonne zugeführt wird.

7. Verfahren nach zumindest einem der vorherigen Ansprüche,
dadurch gekennzeichnet,
daß zur Molekularsiebbehandlung ein Molekularsieb mit 0,4 bis 0,5 nm Porendurchmesser verwendet wird.

8. Verfahren nach zumindest einem der vorherigen Ansprüche,
dadurch gekennzeichnet,
daß die Molekularsiebbehandlung bei Temperaturen bis zu 50 °C erfolgt.

9. Verfahren nach zumindest einem der vorherigen Ansprüche,
dadurch gekennzeichnet,
daß das Verfahren kontinuierlich mit zumindest zwei Adsorbern durchgeführt wird.
